# EUROPÄISCHE PATENTANMELDUNG

(11) **EP 1 125 597 A1**
(43) Veröffentlichungstag der Anmeldung: **22.08.2001**
(21) Anmeldenummer: 01101419.8
(22) Anmeldetag: 23.01.2001
(51) Int. Cl.: A61M 39/16, F16K 7/07

(54) **Dampfsterilisierbares Flüssigkeitsventil**

(30) Priorität: 12.02.2000 DE 20002476 U
(71) Anmelder: B. BRAUN MELSUNGEN AG, 34212 Melsungen (DE)
(72) Erfinder: Schlarb, Alois, Dr., 6110 Wolhusen (CH); Bertschi, Samuel, 6170 Schüpfheim (CH); Marks, Stephan, 6182 Escholzmatt (CH)
(74) Vertreter: Selting, Günther, Dipl.-Ing.

(57) **Zusammenfassung**

Das Flüssigkeitsventil weist eine Ventilscheibe (22) auf, die von einem Dorn (20) zentrisch abgestützt wird und deren Rand (23) gegen einen Ventilsitz (24) drückt. Die Ventilscheibe (22) kann durch einen in eine Kupplungshülse (16) eingeführten Konnektor aufgestoßen werden. Die Kupplungshülse (16) ist vor der Flüssigkeitsentnahme durch eine Kappe (33) verschlossen. Durch Drehen eines Knopfs (39) reißt eine Sollbruchstelle (31), wodurch die Kappe (33) entfernt werden kann. Um während der erforderlichen Dampfsterilisation eine Verschiebung der Ventilscheibe (22) zu verhindern, ist eine Haltevorrichtung vorgesehen, die die Ventilscheibe während der Sterilisation auf dem Dorn (20) hält. Diese Haltevorrichtung besteht aus einer an der Ventilscheibe vorgesehenen Eingriffsvorrichtung (26) und kann einen Niederhalter (28) aufweisen. Eingriffsvorrichtung (26) und Niederhalter (28) verhindern ein radiales Verschieben der Ventilscheibe (22), Niederhalter (28) und Ringhülse (32) verhindern ein axiales Verschieben.

## Beschreibung

Die Erfindung betrifft ein dampfsterilisierbares Flüssigkeitsventil für die Flüssigkeitsentnahme aus einem Behälter, insbesondere aus einem Behälter, der eine sterile Flüssigkeit enthält.

Aus US 5 775 671 ist ein normalerweise geschlossenes Flüssigkeitsventil bekannt, bei dem eine flexible Ventilscheibe von einem zentralen Dorn abgestützt ist und mit ihrem Rand gegen einen ringförmigen Ventilsitz drückt. Das Ventil wird dadurch geöffnet, dass ein Rohransatz eines Entnahmegerätes in die Kupplungshülse des Ventils eingeschoben wird und gegen ein bewegliches Zwischenstück stößt, das seinerseits die Ventilscheibe aufdrückt. Ein ähnliches Flüssigkeitsventil ist in EP 0 928 621 A2 beschrieben. Hierbei weist die Kupplungshülse zusätzlich verschiedene Rastnuten auf, derart, dass der Konnektor wahlweise in einer passiven Stellung angekuppelt werden kann, in der er das Ventil nicht öffnet, und in einer aktiven Stellung, in der er das Ventil aufstößt.

Eine Schwierigkeit der bekannten Flüssigkeitsventile für die Flüssigkeitsentnahme aus Behältern besteht darin, dass sie nach einer Dampfsterilisation nicht die erforderliche Dichtigkeit gewährleisten. Der Behälter, an dem das Flüssigkeitsventil angebracht ist, wird nach dem Befüllen einer Dampfsterilisation ausgesetzt, um sowohl den Behälter als auch die in ihm enthaltene Flüssigkeit zu sterilisieren. Medizinische Flüssigkeiten, wie beispielsweise Spüllösungen, werden in Kunststoffbeuteln verpackt, die mit einem Flüssigkeitsventil versehen sind. Zur Flüssigkeitsentnahme wird ein Siegel des Beutels aufgebrochen, damit das Flüssigkeitsventil zugänglich wird. Dann wird ein Schlauchkonnektor an das Flüssigkeitsventil angekuppelt, wodurch das Flüssigkeitsventil aufgestoßen wird und die Flüssigkeit aus dem Beutel herausläuft. Derartige Beutel, einschließlich des Flüssigkeitsventils sind Einmalartikel, die einfach und kostengünstig hergestellt werden müssen und dabei dennoch die erforderliche Funktionssicherheit haben.

Der Erfindung liegt die Aufgabe zugrunde, einen dampfsterilisierbares Flüssigkeitsventil zu schaffen, das eine Dampfsterilisation aushält ohne an Funktionsfähigkeit einzubüßen. Die Lösung dieser Aufgabe erfolgt erfindungsgemäß mit den im Anspruch 1 angegebenen Merkmalen. Hiernach ist die die Kupplungshülse bedeckende Kappe des Flüssigkeitsventils mit einer Haltevorrichtung versehen, die die Ventilscheibe an dem Dorn gegen radiale Verschiebungen und axiale Verschiebungen in Richtung der Kupplungshülse sichert. Damit wird verhindert, dass die Ventilscheibe sich bei der Dampfsterilisation unbeabsichtigt verschiebt. Die Dampfsterilisation erfolgt bei mindestens 121°C und einem Druck von etwa 2 bar. Untersuchungen haben gezeigt, dass unter diesen Umständen die Gefahr des unkontrollierten Verlagerns der Ventilscheibe besteht. Dies führt zu undichten Ventilen, mit der Folge, dass nach dem Entfernen der Kappe vom Kupplungselement unbeabsichtigt und vorzeitig Flüssigkeit aus dem Beutel ausläuft. Die Haltevorrichtung ist auch im Anschluss an die Sterilisation wirksam. Sie hält die Ventilscheibe während Transport und Lagerung definiert fest. Die Haltevorrichtung weist bei einer bevorzugten Ausführungsform der Erfindung einen Niederhalter auf, der in die Kupplungshülse ragt und verhindert, dass die Ventilscheibe aus der Eingriffsvorrichtung gehoben werden kann. Beim Abnehmen der Kappe wird der Niederhalter mit entfernt. Erst dann wird die Zwangsfixierung der Ventilscheibe aufgehoben.

Gemäß einer vorteilhaften Ausgestaltung der Erfindung ist der Niederhalter mit der Kupplungshülse über eine Sollbruchstelle verbunden. Der Niederhalter ist also einstückig mit dem Kupplungselement ausgebildet. Hierdurch werden Herstellung und Montage vereinfacht. Außerdem kann durch die Sollbruchstelle eine keimdichte Abdichtung der Öffnung der Kupplungshülse auf einfache Weise sichergestellt werden.

Der Niederhalter kann ein Kopfstück aufweisen, welches einrastend in der Kappe enthalten ist. Dies ermöglicht es, die Kappe sehr einfach durch Aufsetzen zu montieren. Andererseits kann die Kappe nicht wieder abgenommen werden, ohne den Niederhalter mitzunehmen. Die Rastung ist also -eine Einwegrastung, die nur das Aufsetzen der Kappe auf das Kopfstück ermöglicht, nicht aber das anschließende Entfernen der Kappe vom Kopfstück.

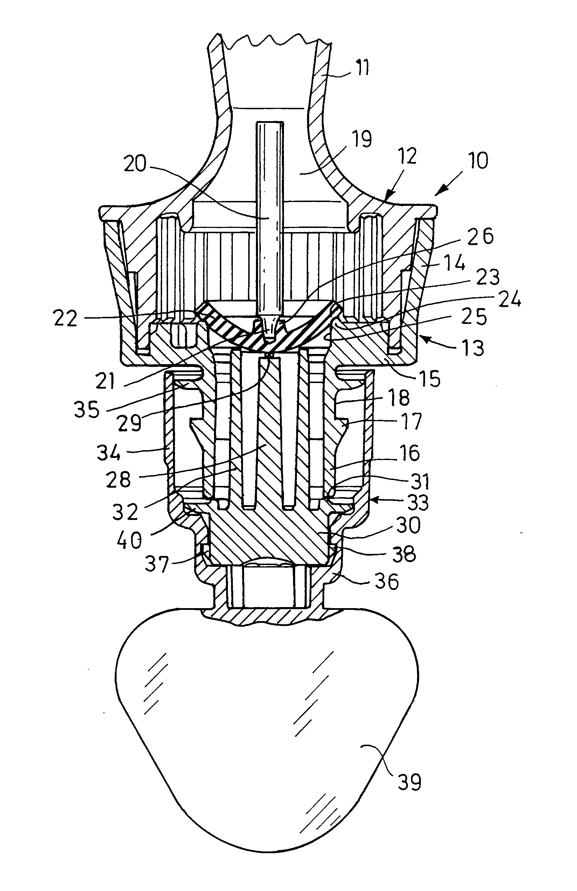

Im folgenden wird unter Bezugnahme auf die einzige Figur der Zeichnung ein bevorzugtes Ausführungsbeispiel der Erfindung näher erläutert.

Die Zeichnung zeigt einen Längsschnitt durch die an einem flüssigkeitsgefüllten Beutel vorgesehene Entnahmevorrichtung.

Die Entnahmevorrichtung ist in der Gebrauchsstellung des Beutels dargestellt, bei der der Beutel mit dem Boden nach oben aufgehängt wird, so dass die Entnahmevorrichtung 10 herabhängt. An die Entnahmevorrichtung kann ein Schlauchkonnektor angekuppelt werden, um Flüssigkeit aus dem Beutel zu entnehmen. Die Entnahmevorrichtung weist einen mit dem (nicht dargestellten) Beutel verbundenen Hals 11 auf, der in das ringförmige Ventilgehäuse 12 übergeht. Auf das Ventilgehäuse 12 ist ein topfartiges Kupplungselement 13 aufgesetzt, das einen Umfangsmantel 14, eine Stirnwand 15 und eine von der Stirnwand abstehende Kupplungshülse 16 aufweist. An der Kupplungshülse 16 befindet sich ein Schnappring 17 von sägezahnförmigem Querschnitt, hinter dem eine Nut 18 für das Einrasten von Rastfingern des zugehörigen (nicht dargestellten) Entnahmekonnektors angeordnet ist.

Das Ventilgehäuse 12 enthält einen Armstern 19, an welchem der axial ausgerichtete Dorn 20 nach vorne absteht. Das vordere Ende des Dornes 20 ist als Spitze 21 ausgebildet. Die Spitze 21 stützt eine Ventilscheibe 22 zentral ab, deren Rand 23 elastisch gegen einen Ventilsitz 24 drückt. Da die Spitze 21 des Dornes in die den Ventilsitz 24 umgebende Öffnung 25 hinein vorsteht, wird die aus Elastomermaterial bestehende Ventilscheibe 22, die im Ursprungszustand eben ist, von der Spitze 21 und dem Ventilsitz 24 becherförmig gebogen. Der Rand 23 der Ventilscheibe drückt abdichtend gegen den ringförmigen Ventilsitz 24.

Die Ventilscheibe 22 weist eine Eingriffsvorrichtung 26 in Form einer ihr zentrisch angeformten rohrförmigen Manschette auf, in die die Spitze 21 des Dornes 20 hineinragt, um die Ventilscheibe zentriert zu halten. Die Eingriffsvorrichtung 26 ist Bestandteil einer Haltevorrichtung zum Festhalten der Ventilscheibe während der Sterilisation.

In der Haltevorrichtung gehört ein Niederhalter 28, welcher zentrisch im Innern der Kupplungshülse 16 verläuft und mit seiner Spitze 29 ein Abheben der Eingriffsvorrichtung von der Spitze 21 des Dornes 20 verhindert.

Der Niederhalter 28 steht von einem Kopfstück 30 ab, welches durch eine umlaufende Sollbruchstelle 31 mit dem Rand der Kupplungshülse 16 verbunden ist. Die Sollbruchstelle 31 besteht aus einer durchgehenden Schwächungslinie, die ein Brechsiegel darstellt. Von dem Kopfstück 30 steht ferner eine Ringhülse 32 ab, die den stabförmigen Niederhalter 28 mit Abstand umgibt und den Zentralbereich der Ventilscheibe 22 ringförmig abstützt. Dadurch wird verhindert, dass die Ventilscheibe in die Öffnung 25 der Kupplungshülse 16 gedrückt werden kann.

Das Kopfstück 30 ist rastend in einer Kappe 33 enthalten, welche mit einem rohrförmigen Mantel 34 die Kupplungshülse 16 umgibt. In der Nähe des Fußes der Kupplungshülse befindet sich ein tellerförmig abstehender Rand 35, der von dem Mantel 34 übergriffen wird und zusammen mit dem Mantel eine Staubdichtung bildet. Von dem Kopfstück 30 steht in der Nähe der Sollbruchstelle 31 ein tellerförmiger Rand 40 ab, der - ebenso wie der Rand 35 - an der Innenwand der Kappe 33 angreift und einen Staubschutz bildet. Auf diese Weise ist die Kupplungshülse 16 von der Kappe 33 staubdicht umschlossen, solange die Sollbruchstelle 31 noch intakt ist. In dem Deckelteil 36 der Kappe 33 befinden sich Rastausnehmungen 37 in Form von Durchbrüchen, die in die Rastklinken 38 des Kopfstücks 30 eingreifen, um das Kopfstück 30 drehfest mit der Kappe 33 zu verbinden. An den Deckelteil 36 der Kappe 33 schließt sich ein Knauf 39 an, der zum Abnehmen der Kappe gedreht wird, wodurch das Kopfstück 30 von der ringförmigen Sollbruchstelle 31 von dem Kupplungselement 13 abreißt. Dann kann die Kappe 33 zusammen mit dem Kopfstück 30 entfernt werden. Danach wird in die Kupplungshülse 16 ein Konnektor eingeschoben, der die Ventilscheibe 22 aufdrückt, so dass Flüssigkeit aus dem Beutel ausfließen kann. Nach dem Entfernen des Konnektors schließt das Ventil selbsttätig wieder.

Die Dampfsterilisation erfolgt bei gefülltem Beutel, während die Kappe 33 auf dem Kupplungselement 13 sitzt. Durch die Dampfbehandlung wird sowohl der Beutelinhalt als auch die Entnahmevorrichtung bzw. das Flüssigkeitsventil sterilisiert. Da bei der thermischen Dampfbehandlung die Ventilscheibe 22 im Zentrum festgehalten und ihre konvexe Seite mehrfach abgestützt wird, können unbeabsichtigte Verschiebungen nicht auftreten. Eingriffsvorrichtung 26 und Niederhalter 28 verhindern ein radiales Verschieben der Ventilscheibe 22, Niederhalter 28 und Ringhülse 32 verhindern ein axiales Verschieben.

## Patentansprüche

1. Dampfsterilisierbares Flüssigkeitsventil für die Flüssigkeitsentnahme aus einem Behälter, mit einem Kupplungselement (13), das eine Kupplungshülse (16) zum Ankuppeln eines Konnektors aufweist, einer flexiblen Ventilscheibe (22), die durch einen zentralen Dorn (20) abgestützt ist und mit ihrem Rand (23) gegen einen Ventilsitz (24) drückt, und einer die Kupplungshülse (16) überdeckenden entfernbaren Kappe (33),
**dadurch gekennzeichnet**,
dass eine die Ventilscheibe (22) auf dem Dorn (20) zentriert haltende Haltevorrichtung vorgesehen ist.

2. Flüssigkeitsventil nach Anspruch 1, dadurch gekennzeichnet, dass die Haltevorrichtung aus einer mit dem Dorn (20) zusammengreifenden Eingriffsvorrichtung (26) der Ventilscheibe (22) besteht.

3. Flüssigkeitsventil nach Anspruch 2, dadurch gekennzeichnet, dass die Haltevorrichtung einen Niederhalter (28) aufweist, der in die Kupplungshülse (16) ragt und die Eingriffsvorrichtung im Eingriffszustand blockiert.

4. Flüssigkeitsventil nach Anspruch 3, dadurch gekennzeichnet, dass der Niederhalter (28) mit der Kupplungshülse (16) über eine Sollbruchstelle (31) verbunden ist.

5. Flüssigkeitsventil nach Anspruch 3 oder 4, dadurch gekennzeichnet, dass der Niederhalter (28) ein Kopfstück (30) aufweist, welches mit der Kappe (33) verbunden ist.

6. Flüssigkeitsventil nach Anspruch 1 oder 2, dadurch gekennzeichnet, dass die Haltevorrichtung eine Ringhülse (32) aufweist, die in die Kupplungshülse (16) ragt und verhindert, dass die Ventilscheibe (22) in die Öffnung (25) der Kupplungshülse (16) gedrückt wird.

7. Flüssigkeitsventil nach Anspruch 6, dadurch gekennzeichnet, dass die Ringhülse (32) mit der Kupplungshülse (16) über eine Sollbruchstelle (31) verbunden ist.

8. Flüssigkeitsventil nach Anspruch 6 oder 7, dadurch gekennzeichnet, dass die Ringhülse (32) ein Kopfstück (30) aufweist, welches mit der Kappe (33) verbunden ist.

9. Flüssigkeitsventil nach einem der Ansprüche 1-8, dadurch gekennzeichnet, dass die Kappe (33) mit einem Knauf (39) versehen ist.

10. Flüssigkeitsventil nach einem der Ansprüche 1-9, dadurch gekennzeichnet, dass die Kupplungshülse (16) einen radial abstehenden Rand (35) aufweist, der die Öffnung der Kappe (33) verschließt.

11. Flüssigkeitsventil nach einem der Ansprüche 5-10, dadurch gekennzeichnet, dass das Kopfstück (30) einen radial abstehenden Rand (40) aufweist, der zusammen mit der Kappe (30) einen die Kupplungshülse (16) schützenden Verschluss bildet.
